# EUROPEAN PATENT APPLICATION

(11) **EP 3 315 099 A1**
(43) Date of publication of application: **02.05.2018**
(21) Application number: 16196293.1
(22) Date of filing: 28.10.2016
(51) Int. Cl.: A61F 2/66

(54) **PROSTHETIC FOOT**

(71) Applicant: Roadrunnerfoot Engineering S.r.l., 20010 Pregnana Milanese (MI) (IT)
(72) Inventor: BONACINI, Daniele, 20151 Milano MI (IT)
(74) Representative: Spina, Alessandro

(57) **Abstract**

A prosthetic foot (100) comprises:
i) a lower lamina (110) having a wavy shape comprising a curved front portion (111) and a curved rear portion (112), whose concavities are oriented upwards with respect to a vertical direction (V), and an intermediate, mostly curved portion (113) having a concavity facing away with respect to the concavities said front and rear portions (111, 112),
ii) a sickle-shaped front lamina (120) comprising a lower end portion (121) restrained to said lower lamina (110) close to its front portion (111), an upper end portion (122) configured so as to allow connection of a mounting member (140) of the prosthetic foot, as well as an intermediate, mainly curved portion (123) arranged between the lower end portion (121) and the upper end portion (122),
iii) a rear lamina (130) comprising an upper portion (131) restrained to the front lamina (120) close to its upper end portion (122), and a lower portion (132) restrained to the lower lamina (110) at its curved rear portion (112).

The overall configuration of the prosthetic foot is such that the three laminas (110, 120, 130) are restrained to each other two by two and that their connection points form the vertices of a triangle.

## Description

### Technical field of the invention

The present invention relates to a prosthetic foot for lower limb amputees, and in particular to a prosthetic foot configured for walking and jogging.

### Background

In order to meet the needs of amputees prosthetic feet have been developed and marketed since the 80s of the last century. Thanks to the use of composite materials such as carbon fiber, these feet can store energy when contacting the ground, during the so-called "loading" step after the initial contact with the ground, and return energy during rolling on the ground, the so-called "mid-stance" step, up to separation from the ground, the so-called "toe-off" step.

Known prosthetic feet for walking are characterized by a ratio between absorbed and returned energy that is lower than or equal to 60%, as well as by a low velocity of elastic response between the loading and the rolling steps, which allows to provide an amputee with a high level of comfort.

An example of a prior art prosthetic foot for walking is described in European patent EP 2090268 B1 in the applicant's name.

Also known are prosthetic feet for running, for example on a running track or on a marathon track. These prosthetic feet are much more rigid than those used for walking and are characterized by a higher ratio between absorbed and returned energy, which is about 80%. Prosthetic feet for running also feature a higher velocity of elastic response.

An example of a prior art prosthetic foot for running is described in European patent EP 2065018 B1 in the applicant's name.

The international publication WO 02/30340 A2 and the publication EP 2644167 A1 disclose a prosthetic foot according to the preamble of the independent claim 1.

An amputee wishing to practice running must then replace the prosthetic foot for walking with a prosthetic foot for running. This implies that the amputee must have both prosthetic feet and is every time required to replace the one with the other one.

### Summary of the invention

The technical problem posed and solved by the present invention is therefore to provide a prosthetic foot which allows to both walk and slow run, in particular jogging, thus overcoming the drawbacks mentioned above with reference to prior art.

This problem is solved by a prosthetic foot according to claim 1.

Preferred features of the present invention are object of the dependent claims.

An idea of solution underlying the invention is to provide a prosthetic foot comprising three laminae that have a predominantly curved shape and are connected to each other two by two by way of fastening means.

More particularly, the prosthetic foot comprises a lower lamina intended to contact the ground, which has two curved ends portions configured to perform the function of a heel and a forefoot of a natural foot, respectively, and an intermediate portion connected between the two curved end portions, which is mainly curved, but features an opposite concavity, and which performs the function of the plantar arch of a natural foot.

The prosthetic foot also comprises a front lamina having the shape of sickle a lower end portion of which is straight and fixed to the lower lamina at the forefoot and an upper end portion of which, also straight shaped, is configured to be connected to an mounting member suitable to allow mounting of the prosthetic foot to other prosthetic components. Between the two end portions an intermediate, mainly curved portion is arranged, that performs the function of a natural ankle.

The prosthetic foot also comprises a rear lamina made up of a straight portion fixed to the front lamina at its curved portion and a curved portion fixed to the lower lamina at the curved end portion, which performs the function of the heel of a natural foot.

The configuration of the prosthetic foot according to the invention is such that during each step of contact with the ground, from the heel contact to the toe-off, the functions of absorbing and returning mechanical energy are performed by at least two laminae simultaneously, which allows to ensure a good level of comfort to an amputee using of the prosthesis, and also to achieve ratios between absorbed and returned mechanical energy in the order of 70-75%, i.e. remarkably higher than those which characterize the known prosthetic feet for walking.

The main advantage offered by the invention is therefore to provide a prosthetic foot which allows to walk, as well as to practice slow running, i.e. jogging.

In other words, the prosthetic foot according to the invention is more versatile than the prosthetic feet known in the art, that are specifically designed for walking or running, but are substantially unsuitable to allow to practice both activities.

The prosthetic foot according to the invention meets the requirements of the standard ISO 10328, but advantageously has a structure that allows it to withstand loads between 2500 N and 3000 N, which are higher than those characterizing prosthetic feet for walking known in the art, and allow to practice jogging.

Further advantages, features and the modes of employ of the present invention will become clear from the following detailed description of some embodiments thereof, given by way of non-limiting examples.

### Brief description of the drawings

Reference will be made to the figures of the accompanying drawings, in which:
- Figure 1 is a perspective view showing a prosthetic foot according to the invention;
- Figure 2 is a side sectional view of the prosthetic foot of figure 1;
- Figures 3, 4 and 5 are respectively a front view, rear and top plan view of the prosthetic foot of figure 1;
- Figures 6, 7, and 8 are side views that schematically show the steps of initial contact with the ground, rolling and toe-off of the prosthetic foot according to the invention;
- Figure 9 is a chart showing a comparison between the vertical forces resulting from the contact with the ground in a natural foot, in a prior art prosthetic foot for walking and in a prosthetic foot according to the invention.

### Detailed description of preferred embodiments

With reference to the figures, a prosthetic foot according to the invention is generally indicated by reference number 100.

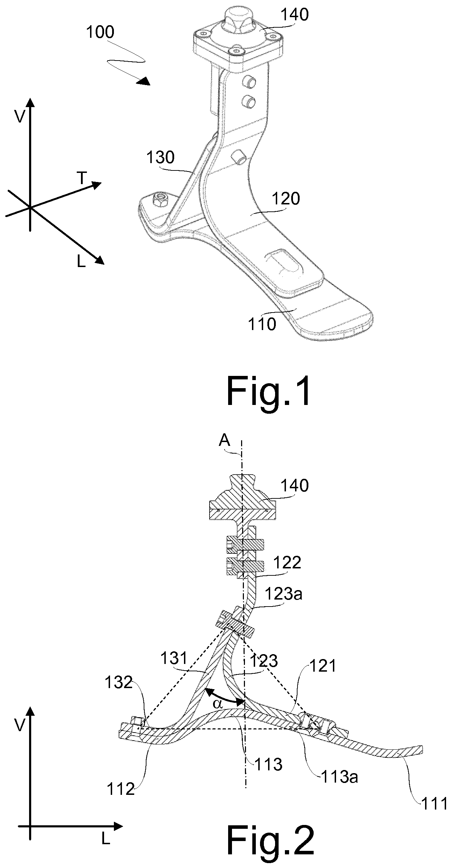

Figure 1 shows the prosthetic foot 100 in a three-dimensional reference system, wherein the prosthetic foot has a length in a longitudinal direction L, which is also the direction of movement relative to a support surface such as the ground, and a height in a vertical direction V, perpendicular to the longitudinal direction L, along which it can be connected to other prosthetic components so as to enable its assembly onto the lower limb of an amputee. The vertical direction V is also the loading direction of the prosthetic foot 100.

The prosthetic foot 100 has width in a transverse direction T, perpendicular to the longitudinal direction L and to the vertical direction V.

The prosthetic foot 100 comprises three laminae connected to each other two by two by way of fastening means. These laminae are made of a multilayer composite material made for example of a plurality of layers of a fabric of carbon fiber, aramid fiber, glass fiber and the like, impregnated with an epoxy resin, an acrylic resin, and the like, that are materials typically used to manufacture prosthetic feet.

A lower lamina 110, intended to contact the ground typically housed in an aesthetic covering having the shape of a human foot in turn received in a shoe, has a wavy shape comprising two curved end portions, a front portion 111 and a rear portion 112 with reference to the longitudinal direction L, respectively, configured to perform the function of the forefoot and of the heel of a natural foot.

As known in the art the curved end portions 111, 112 have respective concavities facing upwards with respect to the vertical direction V, that is, opposite to a supporting surface of the prosthetic foot 100, that allow movements of the prosthetic foot replacing the heel and the forefoot of a natural foot, respectively, during the initial contact with the ground and the toe-off at the end of a rolling or mid-stance step.

An intermediate portion 113 is connected between the curved, front and rear end portions 111 and 112. The intermediate portion 113 is mainly curved and has an opposite concavity in the vertical direction V with respect to the end portions 111, 112. The intermediate portion 113 performs the function of the plantar arch of a natural foot and a vertex thereof passes preferably at or close to a loading straight line A of the prosthetic foot, shown in figure 2.

This configuration of the prosthetic foot 100 allows to obtain a correct orthostatic position, wherein the weight of the amputee is discharged to the ground through the curved front and rear ends of the lower lamina 110, which, as explained above, perform the function of the heel and the forefoot of a natural foot.

The overall length of the lower lamina 110 in the longitudinal direction L approximates the length of a natural foot. It is known that this length varies depending on the user's foot size. The size of a prosthetic foot typically ranges in five classes depending on the shoe size of a user: class I numbers 35-36, class II numbers 37-38, class III numbers 39-40, class IV numbers 41-42, class V numbers 43-44. Also known are prosthetic feet with non-standard size outside the five classes mentioned above.

The prosthetic foot 100 also includes a front lamina 120 that has the shape of a sickle. With reference to the vertical direction V, a lower end straight portion 121 of the front lamina 120 is restrained to the lower lamina 110 on a straight portion 113a of the intermediate portion 113 thereof, that is located close to the forefoot 111. A top end portion 122, that is also straight and substantially vertical, is configured for connection to a mounting member allowing to assemble the prosthetic foot 100 to other prosthetic components. Between the two straight end portions 121, 122 an intermediate portion 123 is connected.

The intermediate portion 123 is mainly curved and performs the function of a natural ankle.

The center of curvature of the intermediate portion 123 is arranged so as to correspond to the center of rotation of a natural ankle. Moreover, the center of curvature is close to the center of gravity of a triangle, whose vertices are arranged at the two contact points of the front and rear ends 111, 112 of the lower lamina 110 with a supporting surface, and at the upper end portion 122 of the front lamina 120. The position of the center of curvature of the intermediate portion 123 in correspondence of the center of gravity of this triangle, which as it is known is a schematic model of the prosthetic foot as a whole, allows to obtain a behavior of the prosthetic foot during rolling that is as similar as possible to the behavior of a natural foot.

With particular reference to figure 2, a connecting portion 123a of the curved portion 123 of the front lamina 120 at its upper end 122 is straight and inclined relative to the vertical direction V of an angle α equal to 25° ± 10°. The straight portion 123a extends diagonally from the intermediate portion 123 toward the top end 122. The technical effect of this configuration is that the curved portion 123 is rotated toward the forefoot area of the prosthetic foot 100, that is forward, thus providing the front lamina 120 with a geometry that is similar to the geometry which characterizes a prosthetic foot for running, such as the prosthetic foot described in the European patent EP 2065018 B1 in the applicant's name. This configuration of the front lamina 120 provides the prosthetic foot with a high propulsion capacity and ensures a plantarflexion higher than that of known prosthetic feet for walking.

More particularly, the intermediate portion 123 has the shape of an arc of a circle that extends preferably for an angle of about 100°, which allows to connect it with the straight portion 123 a inclined at an angle of 25° ± 10°.

The prosthetic foot 100 further comprises a rear lamina 130, in turn comprising an upper, straight portion 131 restrained to the front lamina 120 close to its intermediate portion 123, in particular along the straight portion 123a thereof, and a lower, curved portion 132 restrained to the lower lamina 110 at its curved portion 112, which performs the function of the heel of a natural foot as explained above.

As shown in the figures, the straight portion 131 has the same inclination angle of 25° ± 10° relative to the straight portion 123a of the curved portion 123 of the front lamina 120. Hence the two portions 131, 123 are completely superimposed to each other. This configuration of the rear lamina 130 allows to promote rolling of the prosthetic foot 100, as well as to confer to the front lamina 120 a higher propelling ability than the front lamina of a prosthetic foot for walking as that described in the European patent EP 2090268 B1 in the applicant's name.

Furthermore, the curved portion 112 and the curved portion 132 have the same curvature and are mutually overlapped, thus providing the area of the heel of the prosthetic foot 100 with a higher stiffness than that of a prosthetic foot for walking such as that described in the patent European EP 2090268 B1, wherein the heel is defined solely from the end of the curved lower lamina.

It will be also noted that the straight portion 131 of the rear lamina 130 and the straight portion 123a of the intermediate portion 123 of the front lamina 120, that are superimposed and restrained to each other, define an area having a remarkable rigidity near the mounting member of the prosthetic foot 100. A further area having a remarkable rigidity is that defined by the lower portion of the straight end 121 of the upper lamina 120, which is superimposed and connected to the straight portion 113a of the lower lamina 110.

As schematically shown in figure 2 by way of dashed lines, the connection areas, two by two, between the lower lamina 110, the front lamina 120 and the rear lamina 130 form the vertices of an isosceles triangle whose base extends between the connection areas of the front and rear laminae 120, 130 with the lower lamina 110, whereas the oblique sides extend from such connection areas towards the connection area between the front lamina 120 and the rear lamina 130.

Such an isosceles triangle configuration allows to achieve a high stability, that allows the amputee to achieve a correct orthostatic position, and also represents the portion of the prosthetic foot 100 that is mainly responsible for the accumulation and release of mechanical energy during its use.

Still with reference to figure 2, the connection of the three laminae 110, 120, 130 is made by way of fastening means such as screws and bolts or equivalent, which allows to assemble the three laminae in a simple and fast manner, while ensuring a correct transmission of the loads among them during operation of the prosthetic foot, either when walking or when jogging.

According to an embodiment of the invention, spacers may advantageously be employed in the connection areas between the laminae, for example spacers made of aluminum or carbon fiber, suitable to avoid direct contact between the laminae and to distribute contact pressures.

As mentioned above, the prosthetic foot 100 also includes a mounting member 140 suitable to allow its connection to other prosthetic components, such as tubular attacks and pylons.

The mounting member 140 is restrained to the upper, straight end 122 of the front lamina 120. In particular, the mounting member 140 is restrained behind the rear upper end 122 of the front lamina 120 with respect to the longitudinal direction L. Thanks to this feature, an axis of the mounting member 140, which is also the axis A along which vertical loads area applied to the prosthetic foot 100 when used by an amputee, passes close to or at the vertex of the curved portion 113 of the lower lamina 110, thus contributing to achieve a correct orthostatic position.

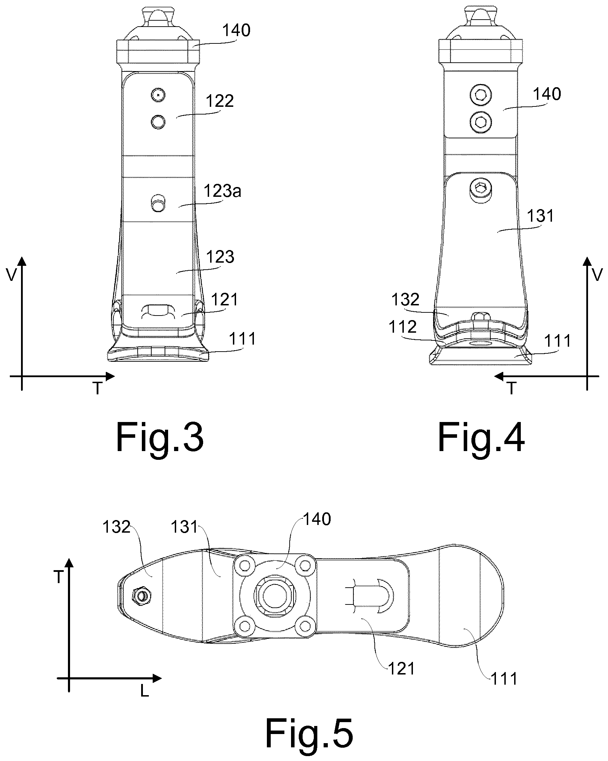

With particular reference to the top plan view figure 5, the lower lamina 110 is thinner at the intermediate portion 113, that serves as the plantar arch, and widens towards the front and rear curved ends 111, 112. This configuration allows to confer to the prosthetic foot a certain twisting ability that allows to simulate pronation of a natural foot.

The front lamina 120, which has the function of storing and returning mechanical energy, instead has a constant width over the whole profile, while the rear lamina 130, which connects the front lamina 120 with the lower lamina 110, has a width increasing from the straight portion 131, fixed to the front lamina 120, to the curved portion 132, fixed to the lower lamina 110.

The thicknesses of the three laminae are preferably variable. In particular, compared to a nominal thickness of each lamina, the front lamina 120 has a greater thickness at the upper end 122, where the mounting member 140 is attached. This has the effect of strengthening the portion through which vertical loads are transferred to the prosthetic foot 100.

The rear lamina 130 has a smaller thickness at the lower end 132 fixed to the lower lamina 110 at its curved portion 112, and the lower lamina 110 has a smaller thickness at its front and rear curved ends 111, 112, which has the effect of making more flexible the areas of the prosthetic foot which perform the function of the heel and forefoot of a natural foot. These localized variations of the thickness of the individual laminae are obtained by varying the number and configuration of the layers that form their multilayer laminar structures.

According to an embodiment of the invention, different lamination modes and sequences of layers for each of the three laminae may be also foreseen according to weight of an amputee and the related level of mobility.

The overall configuration of the prosthetic foot 100 according to the invention is such that at least two of the three laminae simultaneously work at each motion step.

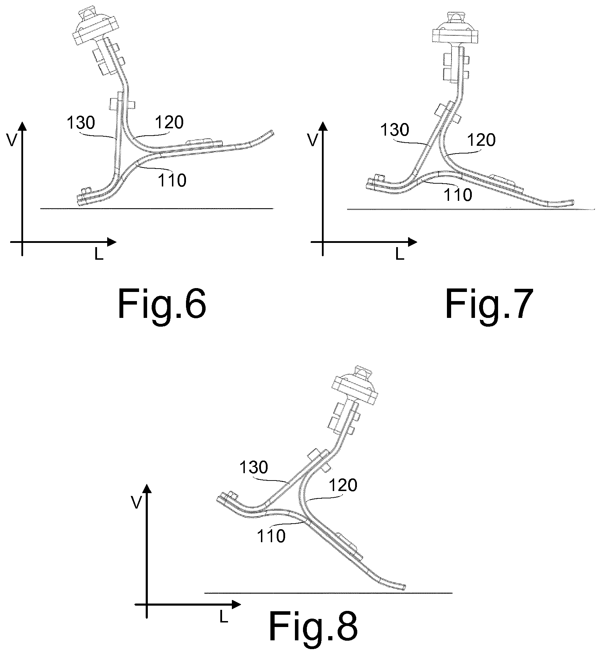

Now referring now to figures 6 to 8, during the phase of initial contact with the ground all the three laminae work in synergy. The lower lamina 110 is mostly responsible for the comfort sensation of the amputee, while deformation of the front lamina 120 and of the rear lamina 130 ensures a gradual ground contact of the forefoot allowing to store mechanical energy in the isosceles triangle portion having vertices at their connection areas.

More particularly, the curved rear end 112 of the lower lamina 110, which defines the heel of the prosthetic foot 100, is elastically deformed thereby absorbing the impact force. Unlike a prosthetic foot for walking as that of the European Patent EP 2090268 B1, in the name of the applicant, the rear lamina 130, fixed to the lower lamina 110, performs the function of the tibialis anterior muscle, thereby controlling and assisting rolling of the prosthetic foot and thus its contact with the ground from the heel to the forefoot.

During the mid-stance step when the curved front and rear ends 111, 112 of the lower lamina 110 are both in contact with the ground, both the front lamina 120 that the rear lamina 130 work synergistically.

After the mid-stance step, the front lamina 120 is deformed and starts to absorb mechanical energy so as to generate a propelling force that allows the prosthetic foot to roll, i.e. to rotate forward in the longitudinal direction L thereby loading the front curved end 111 of the lower lamina 110, serving as the forefoot. During the final, toe-off step, wherein only the curved end 111 of the lower lamina 110 contacts the ground acting as the forefoot, the lower lamina 110 and the front lamina 120 work in a synergistic way.

Unlike a prosthetic foot for walking as that of the European patent EP 2090268 B1, in the name of the applicant, the front lamina 120 performs the function of the Achilles tendon, and hence of the gastrocnemius soleus muscle, which undergoes an eccentric contraction when the foot rolls in the longitudinal direction L in order to stabilize its support in a sagittal plane.

The dorsiflexion of the prosthesis, that allows detachment of the rear end 112 of the lower lamina 110 from the ground and loading of the front end 111, is ensured by the bending of the rear lamina 130 and of the front lamina 120.

In the last step of the support on the ground, the front lamina 120 returns the mechanical energy accumulated in the previous steps thereby promoting the propelling thrust of the front end 111 of the lower lamina 110, i.e. the forefoot of the prosthetic foot 100, which allows to obtain a plantarflexion that is more effective than that of a known prosthetic foot for walking.

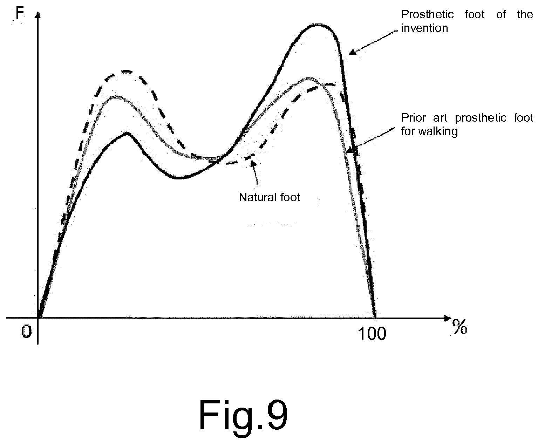

Figure 9 shows a chart of the vertical force progressively discharged by a foot during its contact with the ground from the heel contact to the toe-off. The dashed line curve of the chart particularly shows the typical two-humps curve of a natural foot. It is known that the first hump represents the initially increasing and then decreasing vertical force during the initial contact of the foot with the ground, the force being applied to the ground by the heel. The second hump instead represents the initially increasing and then decreasing vertical force applied to the ground through the forefoot during the propelling step up to the toe-off. These two steps are characterized by the higher vertical loads, while lower vertical loads characterize the mid-stance step, during which the foot rolls from the heel to the forefoot exploiting bending of the plantar arch.

The other two curves show the curves of a prior art prosthetic foot for walking, e.g. according to EP 2090268 B1, and of a prosthetic foot according to the invention, respectively.

As it may be seen, unlike a natural foot and a prior art prosthetic foot for walking, the prosthetic foot according to the invention has a smaller first hump, which indicates a higher absorption ability of the vertical forces at the contact with the ground and therefore a high level of comfort. The second hump is higher than that characterizing a natural foot and a prior art prosthetic foot for walking, which indicates that the prosthetic foot of the invention has a higher propelling ability and a more effective plantarflexion.

Correspondingly, with respect to a natural foot and to a prior art prosthetic foot for walking, a higher amount of mechanical energy, which is about 5-10% higher, is returned, thus promoting propelling particularly in view of using the prosthetic foot for both walking and jogging.

The present invention has been disclosed with reference to preferred embodiments thereof. It will be appreciated that there may be other embodiments based on the same inventive idea, as defined by the scope of protection of the appended claims.

## Claims

1. A prosthetic foot (100) comprising:
i) a lower lamina (110) having a wavy shape comprising a curved front portion (111) and a curved rear portion (112), whose concavities are oriented upwards with respect to a vertical direction (V), and an intermediate, mainly curved portion (113) having a concavity facing away with respect to the concavities said front and rear portions (111, 112),
ii) a sickle-shaped front lamina (120) comprising a lower end portion (121) restrained to said lower lamina (110) close to its front portion (111), an upper end portion (122) configured so as to allow connection of a mounting member (140) of the prosthetic foot, as well as an intermediate, mainly curved portion (123) arranged between the lower end portion (121) and the upper end portion (122),
iii) a rear lamina (130) comprising an upper portion (131) restrained to the front lamina (120) close to its upper end portion (122), and a lower portion (132) restrained to the lower lamina (110) at its curved rear portion (112).
the overall configuration of the prosthetic foot being such that the three laminas (110, 120, 130) are restrained to each other two by two by way of fastening means and that their connection points form the vertices of a triangle.

2. A prosthetic foot (100) according to claim 1, wherein said triangle is an isosceles triangle.

3. A prosthetic foot (100) according to claim 1 or 2, wherein the lower end portion (121) of the front lamina (120) is a straight portion and is restrained to the lower lamina (110) on a straight portion (113a) of the intermediate portion (113) thereof, and wherein the upper portion (131) of the rear lamina (130) is a straight portion and is restrained to a straight portion (123a) connecting the intermediate portion (123) of the front lamina (120) to the upper end portion (122) thereof.

4. A prosthetic foot (100) according to claim 3, wherein the straight portion (123a) connecting the intermediate portion (123) of the front lamina (120) to its upper end portion (122) and the straight upper portion (131) of the rear lamina (130) are inclined by an angle of 25°±10° with respect to said vertical direction (V) and stretch out diagonally from the intermediate portion (123) of the front lamina (120) towards its upper end portion (122).

5. A prosthetic foot (100) according to any one of claims 1 to 4, wherein the center of curvature of the intermediate portion (123) of the front lamina (120) is arranged so as to correspond to the center of rotation of a natural ankle and wherein said center of curvature is close to the center of gravity of a triangle whose vertices are arranged at the contact points of the front and rear ends (111, 112) of the lower lamina (110) with a supporting surface and at the upper end portion (122) of the front lamina (120).

6. A prosthetic foot (100) according to any one of claims 1 to 5, wherein the lower lamina (110), the front lamina (120) and the rear lamina (130) have a variable thickness, and wherein, with respect to a nominal thickness of each lamina, the front lamina (120) has a greater thickness at the upper end portion (122) intended to receive a mounting member (140), the rear lamina (130) has a smaller thickness at the lower end portion (132) restrained to the lower lamina (110) in correspondence with its curved rear portion (112), and the lower lamina (110) has a lower thickness at its curved front and rear portions (111, 112).

7. A prosthetic foot (100) according to any one of claims 1 to 6, further comprising a mounting member (140) configured for connection to other prosthetic components, said mounting member (140) being restrained to the upper end portion (122) of the front lamina (120).

8. A prosthetic foot (100) according to claim 7, wherein said mounting member (140) is restrained behind the upper end portion (122) of the front lamina (120) in the longitudinal direction (L).
